# EUROPEAN PATENT SPECIFICATION

(11) **EP 3 231 296 B1**
(45) Date of publication and mention of the grant of the patent: **24.04.2019**
(21) Application number: 17166146.5
(22) Date of filing: 12.04.2017
(51) Int. Cl.: A23L 33/00, A23L 33/10, A23L 33/115, A23L 33/12, A23L 33/125

(54) **USE OF A COMPOSITION COMPRISING ALPHA-LIPOIC ACID**
VERWENDUNG EINER ZUSAMMENSETZUNG MIT ALPHA-LIPONSÄURE
UTILISATION D'UNE COMPOSITION COMPRENANT DE L'ACIDE ALPHA-LIPOÏQUE

(30) Priority: 14.04.2016 IT UA20162588
(43) Date of publication of application: 18.10.2017
(73) Proprietor: Difass International S.r.l., 47853 Coriano (Rimini) (IT)
(72) Inventor: AGOSTINI, Alida, I-47865 SAN LEO (Rimini) (IT); GIORGINI, Davide, I-47039 SAVIGNANO SUL RUBICONE (Forlì-Cesena) (IT)
(74) Representative: Gerbino, Angelo

(56) References cited:
- EP-A1- 1 214 893
- CN-A- 103 005 204
- US-A1- 2007 196 442

## Description

Alpha-lipoic acid, also called lipoic acid or thioctic acid, is a small organic molecule characterized by the presence of a disulfide group. Two enantiomers of lipoic acid (R or S) exist chemically, which, in most of the commercial preparations, are both present in equal amounts. It is also known that only the R form is found in commercial products.

Lipoic acid is an essential endogenous cofactor of several multi-enzymatic complexes active at the mitochondrial level, and therefore it is important for the production of energy in the organism. It is known to be a metabolic antioxidant, that, due to its amphiphilic properties, is able to perform such action both in the membranes (lipophilic environment) and in the cellular cytosol (hydrophilic environment). It is present in small quantities in red meat, liver, heart, kidneys, spinach, broccoli and tomatoes. The human body synthesizes lipoic acid only in small amounts from fatty acids and cysteine. Being synthesized in the body, lipoic acid is not considered an essential nutrient; however, there may be special conditions, for example, pathological or pre-pathological situations, in which such a compound assumes a conditional essentiality, for example in the case of diabetic neuropathy.

Like other antioxidants, such as glutathione, lipoic acid is part of a redox couple together with its reduced form, dihydrolipoic acid (DHLA). Unlike glutathione, of which only the reduced form is an antioxidant, both the oxidized form and the reduced form of lipoic acid carry out an antioxidant activity. In particular, this activity is carried out by:
- its ability to directly neutralize (scavenger) reactive oxygen species (ROS);
- its ability to regenerate endogenous antioxidants, such as glutathione;
- its chelating activity on metals, resulting in reduced production of ROS.

In the clinical setting, as already mentioned, lipoic acid finds its greatest use in diabetic neuropathies and, more in general, has proven itself useful in treating diabetes mellitus by acting at the level of the metabolic pathways of insulin via the increased uptake of glucose and glycogen synthesis. Its therapeutic activity has been demonstrated by several studies: for example, the SYDNEY 2 study, wherein a daily oral dose of 600 mg improved the symptoms of diabetic polyneuropathy, and the NATHAN study, concluded in 2011, which demonstrated an improvement of neuropathic alterations.

Lipoic acid may play a protective role against the oxidation of LDL and generally against oxidative stress by physically or chemically toxic agents. Other studies have shown the effectiveness of lipoic acid in neurodegenerative conditions; in fact, it has been suggested that lipoic acid is able to slow down cerebral aging.

Faced with such a wide range of applications and directions for use, lipoic acid has, however, some significant problems that make ingesting it difficult and uncomfortable, particularly in preparations in powder or granules to be dissolved in water or other liquid.

Lipoic acid is known to be not very soluble in water, but it is soluble in ethanol, benzene and chloroform, organic solvents not suitable as ingredients in oral preparations. Thus, its poor solubility in water makes it extremely difficult to use this compound in products such as oral solutions or water-dispersible powders, pharmaceutical forms widely used in the pharmaceutical and food industry. Furthermore, lipoic acid, like most sulfur-containing compounds, has poor organoleptic compliance with respect to odor and taste, a notable concern in products to be taken orally, in particular in solutions for drinking or powders to be reconstituted in water. In fact, lipoic acid is describes as having an unpleasant and intense odor and a sour taste, which in English is often described as "pungent taste".

One way to overcome the organoleptic problem of lipoic acid is by parenteral administration of lipoic acid. The patent RU 2451511 describes the production process of the injectable form of a drug based on lipoic acid. The process provides for the formation of lipoic acid salt with N-methylglucamine and the addition of polyethylene glycol, an additional excipient that can exert adverse effects on the body. The injectable form eliminates the organoleptic problem of lipoic acid upstream, but it is an invasive route of administration, with poor compliance and not ideal or desirable for compounds such as lipoic acid.

For the ingestion of oral lipoic acid in products to be reconstituted in water, other forms have been designed to mask the taste to attempt to overcome the problems related to the organoleptic characteristics, such as coating the lipoic acid with food additives, such as talc, ethyl cellulose, gums, etc. The microgranules obtained generate non-homogeneous solutions in water; they tend to settle to the bottom and their presence is felt in the mouth. Although thickening additives are added to the preparations, the ingestion of the lipoic acid mixture and additives reconstituted in water remains complicated by the lack of uniformity in the dispersion of the microgranules, and the risk of incorrect and/or incomplete ingestion of the lipoic acid. Moreover, the simultaneous use of a considerable number of additives leads to a significant increase in cost, not to mention that these compounds generally do not satisfactorily solve the problem related to taste.

The Japanese patent application 2006-315995, published on 24.11.2006, made known the preparation method of the composition of lipoic acid with cyclodextrins, which includes pouring the alcoholic lipoic acid solution slowly into the hydroalcoholic cyclodextrin solution, with subsequent removal of the solvents to obtain a solid compound. The use of cyclodextrins as masking agents leads to improved palatability, in addition to improved dispersibility in water; however, the substantial increase in the price of the final compound makes this composition economically uncompetitive.

Lipoic acid salts, or lipoates, are also known to occur on the market. These, despite showing a better profile of solubility in water than the acid form, may present regulatory problems in use, as lipoates are not clearly marked as accepted sources of lipoic acid. Furthermore, lipoic salts have a characteristic odor and an unpleasant taste, making, their use problematic.

A problem underlying the present invention is therefore to encourage the ingestion of alpha-lipoic acid or its salts contained in preparations or mixtures to be reconstituted in water or other liquids.

According to the invention, the above problem has been solved by the use of a composition comprising alpha-lipoic acid and/or a salt thereof, and at least one soluble fructan, to improve the organoleptic characteristics of alpha-lipoic acid for use in products taken orally.

Fructans are carbohydrates that contain repeated fructose units. Depending on the number of units they contain, fructans are separated into long-chain and short-chain. Inulin is an example of a long-chain fructan, while fructooligosaccharides (FOS) are short-chain fructans. As short-chain fructans, fructooligosaccharides generally have a degree of polymerization from 3 to 5. Fructooligosaccharides are generally used in products such as food integrators for the known therapeutic properties they possess. They are mainly prebiotic fibers and, as such, are resistant to digestion in the gastrointestinal tract. They are fermented by a limited number of bacteria in the colon, mainly bifidobacteria, suggesting a consequent inhibition of the growth of pathogenic bacteria. Anticarcinogenic actions are also attributed to FOS, probably derived from the butyrate resulting from their fermentation, lipid-lowering and hypoglycemic actions, resulting from the fermentation metabolite propionate, as well as being useful in improving mineral absorption and balance. Generally, they are available in food integrators and their dosage varies between 4 and 10 g/day. Although they are composed of units of D-fructose and D-glucose, FOS have a low sweetening power and are not actually used for this purpose.

Surprisingly, it has been found that the combination of FOS with a degree of polymerization (DP) of 3 to 5 - alpha-lipoic acid and/or a salt thereof results in a substantial improvement in the unpleasant odor typical of lipoic acid and it reduces the perception of "pungent taste", which usually occurs in various areas of the oral cavity after administration.

Other soluble fructans, either long-chain or short-chain, may have the same effect, but do not form part of the claimed invention.

"Soluble" here means that the fructan is dissolved essentially instantaneously in water. Furthermore, a dispersion of alpha-lipoic acid has been observed on the surface of the solution, unlike what happens in a solution that does not contain FOS with a DP of 3 to 5, in which the alpha-lipoic acid precipitates to the bottom of the glass, making its ingestion especially difficult.

The invention finds, therefore, a particular use in improving the organoleptic characteristics of alpha-lipoic acid. More particularly, the palatability of alpha-lipoic acid linked to its odor and pungent taste in products for oral use is improved.

Preferably, the FOS with a DP of 3 to 5 are employed in the form of commercially available powders.

Advantageously, the composition for the use of the invention may comprise:
50 to 600 parts by weight of alpha-lipoic acid and/or a salt thereof; and
3000 to 10000 parts by weight of FOS with a DP of 3 to 5.

More preferably, the composition for the use of the invention comprises:
50 to 300 parts by weight of alpha-lipoic acid and/or a salt thereof; and
3000 to 5000 parts by weight of FOS with a DP of 3 to 5.

Even more preferably, the composition for the use of the invention comprises:
100 to 150 parts by weight of alpha-lipoic acid and/or a salt thereof; and
3000 to 4000 parts by weight FOS with a DP of 3 to 5.

The compositions for the use of the present invention may also provide for the use of a granulate formed of alpha-lipoic acid and FOS, achieved by means of fluidized-bed granulation technology, for example according to the following method. First, a mixture of lipoic acid and FOS is prepared by weighing the amount accurately. The powder mixture is then introduced in the apparatus and held in suspension by a continuous air jet. The moving powder is humidified by a water spraying nozzle. Granules are formed by contact of the powder with the water, then the hot air causes the solvent to evaporate and the particles remain welded, forming granules.

In order to achieve an effective action aiding the activity of the alpha-lipoic acid, for the use of the invention, the composition may be used in combination with the other ingredients in effective amounts with beneficial effects on the body, for example, "Whey Protein Isolate" (WPI).

In the present description, the expression "Whey Protein Isolate" is meant to indicate a group of isolated milk whey proteins, obtained through purification processes and spray-drying.

Advantageously, the composition for the use of the invention may also incorporate, for each dosage unit, at least one excipient suitable from a dietetic point of view, in particular an excipient selected from those commonly employed in the field of formulation of food compositions and food integrators. By way of non-limiting example, such excipients may be chosen among those commonly used in the industry, such as those authorized in Regulation (EU) No 1129/2011.

The composition for the use of the invention may also be formulated in solid or liquid form, depending on the nature of the various ingredients used, employing preparative techniques and methods known to those skilled in the art.

The composition for the use of the invention may therefore be advantageously formulated in powder or granules packaged in sachets, bi-compartment sachets or stick packs to be reconstituted or to be taken directly, granules or effervescent tablets, ingestible tablets, chewable tablets, openable capsules, soft capsules, pearls, syrups, solutions, suspensions or oral drops packaged in flacons, vials, stick packs or sachets, selecting, according to the knowledge of those skilled in the art, the various ingredients in the appropriate physical form.

In accordance with a further aspect of the invention, a kit is also made available for the use of the invention comprising:
- a liquid phase acceptable in the dietetic field in a vial, and
- a powder phase in a dosing cap of said vial, said cap containing partially or integrally a composition comprising alpha-lipoic acid and/or a derivative thereof and at least one soluble fructan, and said vial containing the remaining part of this composition.

In a preferred embodiment, the kit for the use of the invention includes a vial with a measured quantity of a liquid phase acceptable from a dietetic point of view and a dosing cap including a predetermined amount of the composition for the use of the invention.

Below are described tests conducted to illustrate that which is described above also quantitatively. First, a quantity of lipoic acid was identified that was sufficient to generate and make distinctly perceptible all the problems mentioned above, as described in example 1.

### EXAMPLE 1

Alpha-lipoic acid in commercially available powder was weighed and dispersed in individual glasses in a fixed quantity of water (25 ml), according to the quantities shown in Table I, obtaining 3 solutions.

**Table I**

| **Solution A (25 ml)** | | **Solution B (25 ml)** | | **Solution C (25 ml)** | |
|---|---|---|---|---|---|
| Ingredient | Quantity (mg) | Ingredient | Quantity (mg) | Ingredient | Quantity (mg) |
| Alpha-lipoic acid | 50 | Alpha-lipoic acid | 100 | Alpha-lipoic acid | 300 |

Each solution was ingested orally in increasing order of quantity of lipoic acid. Between one sample and another, an appropriate amount of time elapsed (at least 1 hour) with rinsing of the mouth, so that the rating of each administration was not influenced by the previous one. After ingesting all the samples, the level/degree of odor and pungent taste of the solutions was evaluated, assigning a score from 0 (no perception) to 5 (highest perception).

For the final sample, the lipoic acid solution which generated a score greater than or equal to 3 was chosen, corresponding to a quantity of lipoic acid equal to 50 mg in 25 ml of water.

Subsequently, additional compositions useful for describing the advantages of the present invention are illustrated in the examples that follow, made with lipoic acid in the presence and absence of FOS.

### EXAMPLE 2

Using a method known per se, two solutions were prepared using commercially available powder ingredients, according to the composition shown in Table II, dissolved in 25 ml of water.

**Table II**

| **Solution A (25 ml)** | | **Solution B (25 ml)** | |
|---|---|---|---|
| Ingredient | Quantity (mg) | Ingredient | Quantity (mg) |
| Alpha-lipoic acid | 50 | Alpha-lipoic acid | 50 |
| Fructooligosaccharides | 0 | Fructooligosaccharides | 1500 |

### EXAMPLE 3

Using a method known per se, two solutions were prepared using commercially available powders ingredients, according to the composition shown in Table III, dissolved in 25 ml of water.

**Table III**

| **Solution A (25 ml)** | | **Solution B (25 ml)** | |
|---|---|---|---|
| Ingredient | Quantity (mg) | Ingredient | Quantity (mg) |
| Alpha-lipoic acid | 50 | Alpha-lipoic acid | 50 |
| | | Fructooligosaccharides | 2000 |

### EXAMPLE 4

### (Evaluation of the organoleptic characteristics)

In order to evaluate and describe the organoleptic characteristics of lipoic acid, in the presence and in the absence of FOS, resulting from the administration of the composition, the following experimental protocol was used.

### General criteria

Each of the compositions referred to in the previous example 2 was tested on a group of persons, consisting of 3 subjects. The inclusion criteria for the admission to the test were exclusively ethical, i.e. a subject had to be of age, healthy and have no taste alterations related to disorders and/or pathologies.

### Methods of testing

The experiment was performed blind. Each person individually tasted first one and then the other solution of the examples, waiting an appropriate amount of time between one tasting and the other (at least 1 hour) and rinsing the mouth so that the evaluation of each administration was not affected by the previous one.

### Evaluation criteria

For each solution tested, a score from 0 to 5 was assigned to the solutions, assessing the dispersion, the odor and the "pungent taste" perceived according to the rating scales listed below.

**Dispersion**

| |
|---|
| 0: precipitated to the bottom of the solution |
| 1 |
| 2 |
| 3 |
| 4 |
| 5: maximum dispersion on the surface of the solution |

**Odor, pungent taste**

| |
|---|
| 0: no perception |
| 1 |
| 2 |
| 3 |
| 4 |
| 5: maximum perception |

### Results

The results obtained are reported in Table IV.

**Table IV**

| **Solution A** | **Solution B** |
|---|---|
| Participant 1 | Participant 1 |
| Dispersion: 0 | Dispersion: 4 |
| Odor: 5 | Odor: 3 |
| Pungent taste: 4 | Pungent taste: 2 |

| Participant 2 | Participant 2 |
|---|---|
| Dispersion: 0 | Dispersion: 4 |
| Odor: 5 | Odor: 3 |
| Pungent taste: 3 | Pungent taste: 3 |

| Participant 3 | Participant 3 |
|---|---|
| Dispersion: 0 | Dispersion: 5 |
| Odor: 5 | Odor: 4 |
| Pungent taste: 3 | Pungent taste: 3 |

An examination of the data collected shows that the particular association of alpha-lipoic acid and FOS in general has led to an improvement of the organoleptic characteristics investigated, with respect to the alpha-lipoic acid solution alone. In particular, a marked improvement was observed with regard to the dispersion and the perception of the odor; less noticeable but partly observed was the improvement of the taste, with a reduced perception of the pungent taste.

In light of these unexpected observations, it was desired to further investigate the association of the object of the invention with a different alpha-lipoic/FOS acid ratio and an increase in the number of participants.

### EXAMPLE 5

### (Evaluation of the organoleptic characteristics with different alpha-lipoic acid/FOS ratio)

In order to evaluate and describe the organoleptic characteristics of lipoic acid in the presence and in the absence of FOS, following the administration of the composition with a different alpha-lipoic acid/FOS ratio, the following experimental protocol was used.

### General criteria

Each of the compositions referred to in the previous example 3 was tested on a group of persons, consisting of 7 subjects. The inclusion criteria for the admission to the test were exclusively ethical, i.e. a subject had to be of age, healthy and have no taste alterations related to disorders and/or pathologies.

### Methods of testing

The experiment was performed blind. Each person individually tasted first one and then the other solution of the example, waiting an appropriate amount of time between one tasting and the other (at least 1 hour) and rinsing the mouth so that the evaluation of each administration was not affected by the previous one.

### Evaluation criteria

For each solution tested, a score from 0 to 5 was assigned to the solutions, assessing the dispersion, the odor and the "pungent taste" perceived, according to the rating scales listed below.

**Dispersion**

| |
|---|
| 0: precipitated to the bottom of the solution |
| 1 |
| 2 |
| 3 |
| 4 |
| 5: maximum dispersion on the surface of the solution |

**Odor, pungent taste**

| |
|---|
| 0: no perception |
| 1 |
| 2 |
| 3 |
| 4 |
| 5: maximum perception |

### Results

The results obtained are reported in Table V.

**Table V**

| **Solution A** | **Solution B** |
|---|---|
| Participant 1 | Participant 1 |
| Dispersion: 0 | Dispersion: 4 |
| Odor: 4 | Odor: 0 |
| Pungent taste: 5 | Pungent taste: 2 |

| Participant 2 | Participant 2 |
|---|---|
| Dispersion: 0 | Dispersion: 5 |
| Odor: 1 | Odor: 0 |
| Pungent taste: 3 | Pungent taste: 3 |

| Participant 3 | Participant 3 |
|---|---|
| Dispersion: 0 | Dispersion: 3 |
| Odor: 2 | Odor: 1 |
| Pungent taste: 3 | Pungent taste: 2 |

| Participant 4 | Participant 4 |
|---|---|
| Dispersion: 0 | Dispersion: 4 |
| Odor: 5 | Odor: 3 |
| Pungent taste: 4 | Pungent taste: 1 |

| Participant 5 | Participant 5 |
|---|---|
| Dispersion: 0 | Dispersion: 5 |
| Odor: 4 | Odor: 3 |
| Pungent taste: 3 | Pungent taste: 3 |

| Participant 6 | Participant 6 |
|---|---|
| Dispersion: 1 | Dispersion: 4 |
| Odor: 4 | Odor: 1 |
| Pungent taste: 4 | Pungent taste: 3 |

| Participant 7 | Participant 7 |
|---|---|
| Dispersion: 0 | Dispersion: 4 |
| Odor: 1 | Odor: 0 |
| Pungent taste: 3 | Pungent taste: 2 |

The examination of the collected data confirms the unexpected improvement of the organoleptic characteristics of the compositions with different alpha-lipoic acid and FOS ratios, compared to those containing only alpha-lipoic acid.

All participants noted the lipoic acid dispersion on the surface of the solution and an improvement of the odor in the solution containing lipoic acid and FOS, characteristics not shown by the solution containing only lipoic acid. 5 out of 7 participants found and partly confirmed an improvement in the perception of pungent taste in the mouth, and, therefore, in a way appreciably more remarkable compared to the previous example, a further refinement of the taste of the alpha-lipoic acid and FOS composition was obtained.

In summary, the simple mixture of lipoic acid and FOS according to this invention shows different odor and taste dispersion characteristics, and significant improvement, compared to lipoic acid alone, without the need to resort to salification processes or the inclusion in macromolecules through the formation of chemical bonds (e.g. cyclodextrins).

Naturally, without altering the principle of the invention, the details of implementation and forms of embodiment may vary widely with respect to those described purely by way of example without thereby departing from the scope of the invention as defined in the appended claims.

## Claims

1. Use of a composition comprising:
alpha-lipoic acid and/or a salt thereof; and
at least one soluble fructan, to improve the organoleptic characteristics of alpha-lipoic acid in products for oral use, wherein said soluble fructan is chosen in the group consisting of fructooligosaccharides having a polymerization degree of 3 to 5.

2. Use according to claim 1, wherein said composition comprises:
50 to 600 parts by weight of alpha-lipoic acid and/or a salt thereof; and
3000 to 10000 parts by weight of at least one soluble fructan.

3. Use according to claim 1, wherein said composition comprises:
50 to 300 parts by weight of alpha-lipoic acid and/or a salt thereof; and
3000 to 5000 parts by weight of at least one soluble fructan.

4. Use according to claim 1, wherein said composition comprises:
100 to 150 parts by weight of alpha-lipoic acid and/or a salt thereof; and
3000 to 4000 parts by weight of at least one soluble fructan.

5. Use according to any one of the previous claims, wherein said composition further comprises at least one excipient acceptable in the dietetic field.

6. Use according to any one of the previous claims, wherein said composition is in solid or liquid form.

7. Use according to claim 6, wherein said composition is in the form of powder or granules in sachet, bi-compartment sachet or stick pack to be reconstituted or to be taken directly, effervescent tablet, ingestible tablet, chewable tablet, openable capsule, soft capsule, pearls, syrup, solution, suspension or oral drops in flacon, vial, stick pack or sachet.

8. Use according to any one of the previous claims, wherein said composition is part of a kit comprising:
- a liquid phase acceptable in the dietetic field in a vial, and
- a powder phase in a dosing cap of said vial, said cap containing partially or integrally said composition and said vial containing the remaining part of said composition.

9. Use according to any one of claims 1 to 7, wherein said composition is part of a food integrator.

10. Use according to any one of claims 1 to 7, wherein said composition is part of a food.

## Patentansprüche

1. Verwendung einer Zusammensetzung mit:
alpha-Liponsäure und/oder einem Salz davon; und
zumindest einem löslichen Fructan, um die organoleptischen Eigenschaften von alpha-Liponsäure in Produkten zur oralen Verwendung zu verbessern, wobei das lösliche Fructan ausgewählt ist aus der Gruppe bestehend aus Fructooligosacchariden mit einem Polymerisationsgrad von 3 bis 5.

2. Verwendung nach Anspruch 1, wobei die Zusammensetzung enthält:
50 bis 600 Gewichtsteile an alpha-Liponsäure und/oder einem Salz davon; und
3000 bis 10000 Gewichtsteile von zumindest einem löslichen Fructan.

3. Verwendung nach Anspruch 1, wobei die Zusammensetzung enthält:
50 bis 300 Gewichtsteile an alpha-Liponsäure und/oder einem Salz davon; und
3000 bis 5000 Gewichtsteile von zumindest einem löslichen Fructan.

4. Verwendung nach Anspruch 1, wobei die Zusammensetzung enthält:
100 bis 150 Gewichtsteile an alpha-Liponsäure und/oder einem Salz davon; und
3000 bis 4000 Gewichtsteile von zumindest einem löslichen Fructan.

5. Verwendung nach einem der vorstehenden Ansprüche, wobei die Zusammensetzung ferner zumindest einen Hilfsstoff enthält, der auf diätetischem Gebiet verträglich ist.

6. Verwendung nach einem der vorstehenden Ansprüche, wobei die Zusammensetzung in fester oder flüssiger Form vorliegt.

7. Verwendung nach Anspruch 6, wobei die Zusammensetzung in der Form von Pulver oder Granulat im Beutel, als zweigeteilter Beutel oder Blisterpäckchen zur Wiederherstellung oder zur direkten Einnahme, als Brausetablette, einnehmbare Tablette, Kautablette, öffenbare Kapsel, Weichkapsel, Perlen, Sirup, Lösung, Suspension oder Tropfen zum Einnehmen im Flakon, Ampulle, Blisterpäckchen oder Beutel vorliegt.

8. Verwendung nach einem der vorstehenden Ansprüche, wobei die Zusammensetzung Teil eines Sets ist, mit:
- einer flüssigen Phase, die auf diätetischem Gebiet in einer Ampulle verträglich ist, und
- einer pulverigen Phase in einer Dosierkappe der Ampulle, wobei die Kappe die Zusammensetzung vollständig oder teilweise enthält, und die Ampulle den restlichen Teil der Zusammensetzung enthält.

9. Verwendung nach einem der Ansprüche 1 bis 7, wobei die Zusammensetzung Teil einer Nahrungsmittelsammelpackung ist.

10. Verwendung nach einem der Ansprüche 1 bis 7, wobei die Zusammensetzung Teil eines Nahrungsmittels ist.

## Revendications

1. Utilisation d'une composition comprenant :
de l'acide alpha-lipoïque et/ou un sel de celui-ci ; et
au moins un fructane soluble, pour améliorer les caractéristiques organoleptiques de l'acide alpha-lipoïque dans des produits destinés à une utilisation orale, dans laquelle ledit fructane soluble est choisi dans le groupe constitué par les fructooligosaccharides présentant un degré de polymérisation de 3 à 5.

2. Utilisation selon la revendication 1, dans laquelle ladite composition comprend :
50 à 600 parties en poids d'acide alpha-lipoïque et/ou d'un sel de celui-ci ; et
3 000 à 10 000 parties en poids d'au moins un fructane soluble.

3. Utilisation selon la revendication 1, dans laquelle ladite composition comprend :
50 à 300 parties en poids d'acide alpha-lipoïque et/ou d'un sel de celui-ci ; et
3 000 à 5 000 parties en poids d'au moins un fructane soluble.

4. Utilisation selon la revendication 1, dans laquelle ladite composition comprend :
100 à 150 parties en poids d'acide alpha-lipoïque et/ou d'un sel de celui-ci ; et
3 000 à 4 000 parties en poids d'au moins un fructane soluble.

5. Utilisation selon l'une quelconque des revendications précédentes, dans laquelle ladite composition comprend en outre au moins un excipient acceptable dans le domaine diététique.

6. Utilisation selon l'une quelconque des revendications précédentes, dans laquelle ladite composition est sous forme solide ou liquide.

7. Utilisation selon la revendication 6, dans laquelle ladite composition est sous la forme de poudre ou de granulés dans un sachet, un sachet à deux compartiments ou un sachet stick à reconstituer ou à prendre directement, de comprimé effervescent, de comprimé ingérable, de comprimé à mâcher, de capsule ouvrable, de capsule molle, de perles, de sirop, de solution, de suspension ou de gouttes orales dans un flacon, une fiole, un sachet stick ou un sachet.

8. Utilisation selon l'une quelconque des revendications précédentes, dans laquelle ladite composition fait partie d'un kit comprenant :
- une phase liquide acceptable dans le domaine diététique dans une fiole, et
- une phase pulvérulente dans un bouchon doseur de ladite fiole, ledit bouchon contenant partiellement ou intégralement ladite composition et ladite fiole contenant le reste de ladite composition.

9. Utilisation selon l'une quelconque des revendications 1 à 7, dans laquelle ladite composition fait partie d'un intégrateur alimentaire.

10. Utilisation selon l'une quelconque des revendications 1 à 7, dans laquelle ladite composition fait partie d'un aliment.
